# NOUVEAU FASCICULE DE BREVET EUROPEEN

(11) **EP 1 604 025 B2**
(45) Date de publication et mention de la décision concernant l'opposition: **03.10.2018**
(45) Mention de la délivrance du brevet: 15.07.2009
(21) Numéro de dépôt: 04720033.2
(22) Date de dépôt: 12.03.2004
(51) Int. Cl.: C12N 15/31

(54) **BACTERIES LACTIQUES TEXTURANTES**
KONSISTENZ VERLEIHENDE MILCHSÄUREBAKTERIEN
TEXTURIZING LACTIC BACTERIA

(30) Priorité: 17.03.2003 FR 0303242
(43) Date de publication de la demande: 14.12.2005
(73) Titulaire: DuPont Nutrition Biosciences ApS, 1411 Copenhagen K (DK)
(72) Inventeur: Horvath, Philippe, 86140 Scorbe-Clairvaux (FR); Manoury, Elise, 86100 Chatellerault (FR); Huppert, Sonia, 94300 Vincennes (FR); Fremaux, Christophe, 86000 Poitiers (FR)
(74) Mandataire: D Young & Co LLP
(86) Numéro de dépôt international: PCT/FR2004/000610
(87) Numéro de publication internationale: WO 2004/085607

(56) Documents cités:
- EP-A2- 0 331 564
- HU-B1- 224 134
- BROADBENT J R ET AL: "Biochemistry, genetics, and applications of exopolysaccharide production in Streptococcus thermophilus: a review." JOURNAL OF DAIRY SCIENCE. UNITED STATES FEB 2003, vol. 86, no. 2, février 2003 (2003-02), pages 407-423, XP009018436 ISSN: 0022-0302
- DATABASE EMBL [Online] EBI; 4 janvier 2002 (2002-01-04), PLUVINET A.: "Streptococcus thermophilus eps locus" XP002256424 Database accession no. AJ289861
- DATABASE EMBL [Online] EBI; 3 août 1999 (1999-08-03), GUEDON, G. ET AL.: "Streptococcus thermophilus variable locus including exopolysaccharide synthesis genes and insertion sequences." XP002256425 Database accession no. Z98171
- DATABASE EMBL [Online] EBI; 2 avril 2001 (2001-04-02), KNEIDINGER B. ET AL.: "Aneurinibacillus thermoaerophilus putative sugar transferase gene, partial cds." XP002256426 Database accession no. AF324836
- DATABASE EMBL [Online] EBI; 21 octobre 1999 (1999-10-21), LLULL D. ET AL.: "Streptococcus pneumoniae cap37 locus" XP002256427 Database accession no. AJ131984
- DATABASE EMBL [Online] EBI; 7 novembre 2002 (2002-11-07), RALLU F. ET AL.: "Streptococcus thermophilus eps type VII operon and flanking sequence." XP002256428 Database accession no. AF454498
- DATABASE GENSEQ [Online] DERWENT; 21 février 2002 (2002-02-21), RALLU F. ET AL.: "Streptococcus thermophilus eps10 operon #2." XP002256429 Database accession no. ABA01447 & WO 01/79500 A (INRA/DANONE/RHODIA) 25 octobre 2001 (2001-10-25)
- DATABASE EMBL [Online] EBI; 1 février 1999 (1999-02-01), IANNELLI F. ET AL.: "Streptococcus pneumoniae DexB (dexB) gene, partial cds." XP002256430 Database accession no. AF026471
- Certified translation of specification of D1
- Alignment of sequence of Prebiolact-2 deposited as NCAIM(P)B 001284 (line 2) with SEQ ID NO: 1 of opposed patent (line1)
- Deposit Receipt for Prebiolact-2 deposited as NCAIM(P)B 001284 on 22.02.2000
- First Declaration of Dr. Andras Unger, Director of HDRI
- Second Declaration of Dr. Andras Unger, Director of HDRI
- Delivery note relating to sale A of Prebiolact-2 by HDRI to Nutricia (copy of original and English translation)
- Invoice no. 1055 relating to sale A of Prebiolact-2 by HDRI to Nutricia (copy of original and English translation)
- Banc extract showing payment of invoice no. 1055 by Nutricia to HDRI (copy of original and English translation)
- Delivery note relating to sale B of Prebiolact-2 by HDRI to Nutricia (copy of original and English translation)
- Invoice no. 1299 relating to sale B of Prebiolact-2 by HDRI to Nutricia (copy of original and English translation)
- Banc extract showing payment of invoice no. 1299 bx Nutricia to HDRI (copy of original and English translation)
- Invoice no. 122001259 relating to sale of 90 cartons Prebiolact-2 by HANSEN to HDRI
- Extract of accounting records showing payment of invoice no. 122001259 by HDRI to HANSEN
- Declaration of Anette Wind
- Excerpt of results of Google search
- BATT C.A. ET AL: 'Encyclopedia of Food Microbiology', 2000, ACADEMIC PRESS, SAN DIEGO pages 2127 - 2133

### Liste de séquences

<110> RHODIA CHIMIE
<120> Bactéries lactiques texturantes
<130> P 02244
<160> 8
<170> PatentIn version 3.1
<210> 1
   <211> 16032
   <212> ADN
   <213> Streptococcus thermophilus
<400> 1
<210> 2
   <211> 906
   <212> ADN
   <213> Streptococcus thermophilus
<400> 2
   aaggagagat tcgtgaaacc taaaatactt gtacttatgg caacctataa tggaggaaaa      60
   tacttacgag agcaattaga tagtgttttt ttacaaaaaa atgtggatat tactgtcctg     120
   gttcgtgacg atggttcaat tgacaatacg tgtgctattc ttgatgaata tagtaagaga     180
   tacaacttaa tatggtattc agggcaacat ctaaatgttg cgaatggatt ttataatttg     240
   atggaagaag cagtcaaaat ggattttgac tacttcgctt tctgtgatca ggatgatgtt     300
   tgggatacag ataaactttc catcggagtt tctgcaattt gtagttttaa tgaacctgct     360
   ttatattatt gtggtcaaag attggccgac ggtaatttaa attttatagc aaatcatgaa     420
   ctcaacacag aacggacact ccatacaagg tttatccttt ctgattttgc tggttgtaca     480
   ggagttttca ataaatcgtt attaaaggaa gttgttagct atagaccgaa gtatatgttg     540
   atgcatgata catggatatt aaaagtctgt ttggctctag gtggacaggt tattattgat     600
   acaagaccgc atatgtatta taggcaacat ggtggtaata cagttggtct tggaagaagt     660
   ctatctgcat acttcaaaca ggtaaaacag tatataactg aatataaagt ggaaccacag     720
   atgaaggaac ttctcttagg atatggtgat agaattattt cggagtatgc tgtaattgca     780
   aatgcttgtt gtaattataa aacggatcgt gaggcacgta cttttttatt aaatagaaat     840
   aatgttgatt tttgcacgat gggattaaat atcaccttta aattaaaagt tctattaaat     900
   aaatta                                                                906
<210> 3
   <211> 1032
   <212> ADN
   <213> Streptococcus thermophilus
<400> 3
   atggaggaag agtttttgct ttttagtatt ataatacctg tttttaatgt cgaaaaatat      60
   cttgaggagt gccttctctc gattataggg cagactgatg aaatatctga aggatgtgaa     120
   ataattctta ttaatgacgg gtcaacagat gatagtgcga aaatttgtga tgagtatgag     180
   cgaaaatatc ctgaattgat aaaggtattt cataggtcaa atcatggttt gcttcttaca     240
   cgaaggtttg gatatcaaca tgccgtagga agatacataa ttaattgtga ctcagatgat     300
   tcgttggaac cgattgcact tgaggtgctc aaagacgcaa tcgtcgaata caacaaccca     360
   gatgttatta tatttaacca caatacatat aaaaatggtg taaaggagat tgcatacaat     420
   aacattttta cagagtctgt ttctgcccag atagataagc tagatgtgtt gaaagagttt     480
   ctgactggaa atcgtattaa tagcgtttgt ggtgcaattt gtaaaaagga ttgtattgat     540
   ataaatagag actataataa atataaaggt cttaataatg gagaagatag ccttcaaaag     600
   attgaacaat tcaaaagtgc gaacacgttt gcatatataa ataaacctct ctataattat     660
   agggcaggat cgggtatgac cacaaaattt gatcgaaatt attttaaatc ttttagaata     720
   gttctggatg aaattcaaaa agaacgagaa atgtgggatt ttcctgatgt agatagatat     780
   ttttcaatta aagttttgtc tattgctgga agagcaatca cacagtcaag atacaattgt     840
   tgggagaata aaagcgagca aataagatac ttgcatgata ttcgtaatga tgactacttt     900
   aaaaaagcag tgcttcaatt ggctacaata aggaaatatc ttcagaaaga ccatatttta     960
   ctaataaaga tgttggataa gggtttgatt cgtcttatag ttttgatgtt ggatattaaa    1020
   aataagatag gc                                                        1032
<210> 4
   <211> 1140
   <212> ADN
   <213> Streptococcus thermophilus
<400> 4
   atgaatagta tagataagaa gtatcgtatt aaacttgata cgttaatatt tttgattttt     60
   attatgttag caactttcaa ctattcgatg gagttatcaa tagttggaaa tccattcagg    120
   caagtgattc ttgtactgat gctcatatta ttgttgttta caatcatcct taagaaatac    180
   acaataaaaa aaatgattgt atttattacg gctatagtgt atggattagt taactatcga    240
   gtttcaggtt atacagactt atttatttta ttgcttgcgg cgtatgttgc ggatcaagta    300
   gattttaata acgtgttaaa agttctattt tgggaaaaat tagtgatatt tttatcgctt    360
   aatatatttt cagtgcttgg tgtaatagag atgactaaat tctccatcaa taagtatcta    420
   tccattgtag aagcatatgc tccaggatat gtttcttcga atgtatatgg ctgccaagca    480
   ggagttttat ttttacttta tttatcaatt aatcggtata aattgacaaa gttaaaagtt    540
   ctattagttt ggtttttgag tgttttagta tacattattt gtagatcaag gacagagcta    600
   atcttagtat cagtaacaac agttctttta ttaatatgta ataatccaaa aaactttaat    660
   aaagttaaga ggatactctc gtggggatat ccatctattt tggttttaaa ttttgggtta    720
   atatatactt tttcaatact cggttacggt aatcccatta tgagcactgt aaatgatgta    780
   ttattcaatg ggagaattgg cttggctctc atgaatttta atacttatgg tatttcatta    840
   tttggctccc caattgatgt ttcgatagta gctaaaacga atagatatta tgctttagat    900
   aatggttata cagttctcgt tttatattac ggcctaatag gattattatg gtattcatat    960
   attcaaattc agactgcaaa aaaattagag aagataaatg agttggtcct gatggttgta   1020
   ctctttatca tcaatatctg ggggatttat gaaggaaaca tggtttcttt aggtggaaat   1080
   ttcatgatta tagcttttct ttctaaagta acagataatt attcagaaga attaaaaagt   1140
<210> 5
   <211> 810
   <212> ADN
   <213> Streptococcus thermophilus
<400> 5
   tatatgatac caaaggttat tcactattgt tggtttggag gaaaacctct accagaagat     60
   gcaaaaatgt atatcgagtc atggaaaaaa tatttaccga attatgagat aaaagagtgg    120
   aatgaaacaa actttgattt gaattgttgc gattatgtta aagaggcata tcaagaaaag    180
   aaatgggctt ttgtttcgga ttatgcaaga ctatggatta tttataatta cggtggaatt    240
   tattttgata ctgatgtcga gctaataagt tctcctgaac caatcataga aaatggagct    300
   tttttaggat gtgaactcaa tttcgaaaag gaattatcag gtgatactaa ccaaactgaa    360
   cttgttaacc caggtctagg ttttggtgcg gaaccgaaga aggattttta tcgtgagcta    420
   ttaaatttat acgaatcaca acactttatt gataaaaatg gagacctcaa tttggaaaca    480
   atagtagtga aaacaactag actgttgaag gggcatggtt ttacaggtaa tgattctatt    540
   gaacgagttg caggaattaa tatttactct catgaatatt tttgcccaat taactatcgt    600
   acaggtagaa tttcagtcac tgaaaaaact gtatctatac accattacca agaaagttgg    660
   ctttcggctt tagataagat tattaataag atagaacaga gtagaagtgg agttggaact    720
   cttgaataca aagtcagaag aattgtttct aagccattta gaggagttaa tcgtctgaag    780
   aagaagggaa ttttgggatt tctaaaaaaa                                    810
<210> 6
   <211> 750
   <212> ADN
   <213> Streptococcus thermophilus
<400> 6
   gttcttccta atgatggttt ggttgctttt cattatagtt cttattggac gttttgtctg 60
   cattcaaaaa gctctataat ctctacagta gttttaccca ctatagaaat tatagagccg 120
   aaaaaaatac tgtccaaggc taaggtgcct atcaaatcaa aaatgtccat agaagaagcg 180
   tattcgcatt taaatgcttg tagtccgaga cccaatggga cttgttatac tatgaacaaa 240
   atagaaaatg catatgattt gcaaattatc attgcggcct ataataccga acaatatata 300
   aaagattgct tgaattctgt ctgtggtcaa cattcaaaat ataaaacttt ggtgacagtt 360
   ataaatgatg gttcaacaga tggaacagag catatacttg ctggtataac ttcggaaaat 420
   aggggtggaa gcgagctatg catcagagtt ataaatcagg acaaccgagg gctttcgggt 480
   gccagaaatg ctgctctttc taatattgaa gcaaagtatg taatgttttt agactcagat 540
   gacattctcc cagagaatac tatttctgtt atgttagata aagcatttat aactgatgca 600
   gatattttac aagaaagttg gtattcgttt gatgatgaaa atgtgagtga aaacattctg 660
   gaggaaaaag tttttgaagt tggaacaaaa ggatatgttt cgggatatcc ttggggtaaa 720
   ctatacaaaa gttctgtatt aaaaaatttt 750
<210> 7
   <211> 435
   <212> ADN
   <213> Streptococcus thermophilus
<400> 7
   tttccaaaag gatactggtt tgaagataca ccaatcactt ttattcttgc agcaatgcca 60
   attaaagtgg taaccataaa ggatattgtt tatggctatc gactgaatcc aaatggaatt 120
   acagcaaagt caatctttca taagaaatca gttgatactt tttgggttac ggaattatgt 180
   ttgaacgaac taccaagatt caaagtgtca tatgatcagc aatcatatga atatttatta 240
   agacaatcat tgatgaatga agccaggata aagaatcagg gaaagaaaat acgagaagca 300
   gtatttgtcc tgacatcaga attaatgaaa atttatttta gtggtttttc ttcaaaagat 360
   tcaaagatga agaaaataga aatagcactt agaaatagac aatttatcca gtttgaatta 420
   tcaaaactgt atttg 435
<210> 8
   <211> 1452
   <212> ADN
   <213> Streptococcus thermophilus
<400> 8
   gtgagacagg gagaacagtt agtgaataga tacagatatt tgttaaaaaa cataggcctt     60
   ttgaccctaa gtagtttttc aaccaaatta cttagtttct ttttggtacc gttatatacc    120
   aatatattat cgactactga atatggaacc tatgatttat ttaatactac aataggtgta    180
   ttactcccga tcctcacatt gaatatacag gaagcggtga tgagattttc cattgacagt    240
   aagtatgata gaaaatcaat cgtgacagtg acagcaaggt tctttattct ttcaaattta    300
   attgttattc ttgggctttg ggtaaattat acatttgtat ttagcgtgat tgccaagcag    360
   tatgccatat ttttcttcct gatgttttta tcgcagtcgc tatccgggat gataaccatg    420
   tatgtcagag gaatagacaa aatatctgac ttgtcttttt caagtgtaat agcatctgtt    480
   attactatat gcttaaatgt ttcgttctta gcatttcttc actggggact tgttggatat    540
   tttatggcca atataatcgg cccgatggtt caatcacttt atttgattgt taaagcacat    600
   attttagggg atattcattt agagcaagca tatcagtctg aaaaaaatga aatggttaat    660
   tatagtaaac cattgattgc aaacagcatc gcgtggtggg taaataattt ttctgacaga    720
   tatatcgttg tgattttttg tgggttggct gaaaatggaa tatattcagt ggcatcgaaa    780
   attccttcga tacttaatat ttttcagaca atttttaatc aggcgtggac gctgtctgct    840
   gtaaaggatt tcgaccctga ggataaaaac ggctttttca ccaatacata taaagcatat    900
   aattgcatga tggttgttct atgctctgga attattgtat ttaacaaact gttagccagt    960
   tttttgtatg caaaagactt ttatgttgct tggaaatacg taccatggtt aacaattgca   1020
   atcgtattcg gagcaatgtc cggatatatt ggaggaattt ttgctgctgt caaggattct   1080
   aaaatatttg cgaaatcaac tgtttgtgga gcgataacta atgtaatatt aaatttaata   1140
   cttacaccca ttatgggacc attgggagct gctattgcta cggcagtatc ctattttgag   1200
   gtatggattt ttagatattt gcaatccagg agatatatta ggattagggt taatatattt   1260
   agggatctta ttacttattt cttgctgttt tgtcagtcga ttatactttt gattgaaatg   1320
   gaaaattctt atttatatgt tcttgaaatc ggaattttta ttttgatagt tttgctatat   1380
   tttaaggata ttcttttgct attgaacaaa ggtagtaatt caatcaaaat gaagaaaagg   1440
   ggaagtgaat ta                                                       1452

## Description

La présente invention a pour objet la souche de *Streptococcus thermophilus* déposée le 26 février 2003 à la CNCM sous le numéro I-2980, son utilisation ainsi que l'utilisation d'une souche de bactérie lactique du genre *Streptococcus* comprenant au moins une séquence sélectionnée parmi le groupe constitué par les séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8 pour fabriquer un produit alimentaire ou un ingrédient alimentaire pour texturer un produit alimentaire ainsi qu'un procédé de construction de ces souches.

L'industrie alimentaire utilise de nombreuses bactéries, sous forme notamment de ferments, en particulier des bactéries lactiques, afin d'améliorer la saveur et la texture des aliments mais aussi pour allonger la durée de conservation de ces aliments. Dans le cadre de l'industrie laitière, les bactéries lactiques sont utilisées intensivement afin de provoquer l'acidification du lait (par fermentation) mais aussi afin de texturer le produit dans lesquelles elles sont incorporées.

Parmi les bactéries lactiques utilisées dans l'industrie alimentaire, on peut citer les genres *Streptococcus, Lactococcus, Lactobacillus, Leuconostoc, Pediococcus* et *Bifidobacterium.*

Les bactéries lactiques de l'espèce *Streptococcus thermophilus* sont utilisées de façon extensive seules ou en association avec d'autres bactéries pour la fabrication de produits alimentaires, notamment de produits fermentés. Elles entrent en particulier dans la formulation des ferments utilisés pour la fabrication de laits fermentés, par exemple des yaourts. Certaines d'entre elles jouent un rôle prépondérant dans l'élaboration de la texture du produit fermenté. Cette particularité est étroitement liée à la production de polysaccharides. Parmi les souches de *Streptococcus thermophilus* on peut distinguer des souches texturantes et des souches non texturantes.

On entend par souche de *Streptococcus thermophilus* texturante une souche qui génère des laits fermentés ayant, dans les conditions décrites en exemple, une viscosité supérieure à environ 35 Pa.s, une aire de thixotropie inférieure à environ 2000 Pa/s et un seuil d'écoulement inférieur à environ 14 Pa. On peut définir une souche de *Streptococcus thermophilus* fortement texturante en ce qu'elle génère des laits fermentés ayant, dans les conditions décrites en exemple, une viscosité supérieure à environ 50 Pa.s, une aire de thixotropie inférieure à environ 1000 Pa/s et un seuil d'écoulement inférieur à environ 10 Pa.

Afin de répondre aux exigences des industriels, il est devenu nécessaire de proposer de nouvelles souches texturantes de bactéries lactiques, notamment de *Streptococcus thermophilus.*

Aussi le problème que se propose de résoudre l'invention est de fournir une souche de bactérie lactique ayant de bonnes propriétés de texturation des produits alimentaires.

Dans ce but, l'invention propose la souche de *Streptococcus thermophilus* déposée le 26 février 2003 à la CNCM sous le numéro I-2980, son utilisation ainsi que l'utilisation d'une souche de bactérie lactique du genre *Streptococcus* pour fabriquer un produit alimentaire ou un ingrédient alimentaire pour texturer un produit alimentaire.

L'invention propose une nouvelle souche de *Streptococcus thermophilus* déposée le 26 février 2003 à la Collection Nationale de Cultures de Microorganismes sous le numéro I-2980.

L'invention a également pour objet les séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8, ainsi qu'un acide nucléique comprenant au moins une de ces séquences nucléotidiques.

L'invention a également pour autre objet les plasmides, vecteurs de clonage et/ou d'expression comprenant au moins une des séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8, ainsi qu'un acide nucléique comprenant au moins une de ces séquences nucléotidiques.

L'invention s'étend aussi à des bactéries hôtes transformées par un vecteur de clonage ou d'expression ou un plasmide de clonage ou d'expression comprenant la séquence nucléotidique SEQ ID n° 1.

L'invention concerne également le procédé de construction des souches décrites ci-dessus caractérisé en ce que les souches sont obtenues par transformation à l'aide d'un plasmide ou d'un vecteur comprenant au moins une des séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8, ou avec un vecteur comprenant un acide nucléique comprenant au moins une de ces séquences nucléotidiques.

L'invention propose également des compositions bactériennes comprenant au moins une souche décrite ci-dessus. Des compositions alimentaires ou pharmaceutiques comprenant au moins une souche selon l'invention ou au moins une souche obtenue selon le procédé de l'invention ou la composition bactérienne selon l'invention sont également décrites.

La présente invention possède de nombreux avantages en terme de texturation des milieux dans lesquels elle est incorporée. Notamment elle permet d'obtenir des gels, à partir par exemple de laits fermentés, qui sont épais, collants, nappants, filants et résistants au brassage et qui ne sont pas granuleux.

D'autres avantages et caractéristiques de l'invention apparaîtront clairement à la lecture de la description et des exemples donnés à titre purement illustratifs et non limitatifs qui vont suivre.

Les bactéries lactiques sont des procaryotes à Gram-positif qui appartiennent au groupe taxinomique des Firmicutes. Elles sont hétérotrophes et chimio-organotrophes ; généralement anaérobies ou aérotolérantes, leur métabolisme peut être homo- ou hétéro-fermentaire : les bactéries lactiques produisent essentiellement de l'acide lactique par fermentation d'un substrat glucidique. Dépourvues de catalase, les bactéries lactiques constituent un groupe hétérogène de bactéries en forme de coques pour les genres *Aerococcus, Enterococcus, Lactococcus, Leuconostoc, Oenococcus, Pediococcus, Streptococcus, Tetragenococcus, Vagococcus* et *Weissella,* ou en forme de bâtonnets pour les genres *Lactobacillus* et *Carnobacterium.* L'appellation de bactérie lactique est souvent étendue à d'autres bactéries apparentées, telles que *Bifidobacterium.*

Parmi les souches de bactéries lactiques qui conviennent à la présente invention on peut citer les genres *Streptococcus, Lactococcus, Lactobacillus, Leuconostoc, Pediococcus* et *Bifidobacterium.*

La souche de bactérie préférée selon l'invention est *Streptococcus thermophilus.*

*Streptococcus thermophilus* est une espèce naturellement présente dans le lait et largement utilisée dans l'industrie alimentaire, et notamment laitière car elle permet d'acidifier et de texturer le lait. Il s'agit d'une bactérie thermophile homofermentaire.

L'invention concerne tout d'abord la souche de Streptococcus thermophilus déposée le 26 février 2003 à la Collection Nationale de Cultures de Microorganismes sous le numéro I-2980.

L'invention a également pour objet les séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8.

Les séquences nucléotidiques SEQ ID n° 2 à SEQ ID n° 8 font partie d'un opéron d'environ 14350 paires de bases impliqué dans la synthèse de polysaccharides (PS). Cet opéron est compris dans la séquence SEQ ID n° 1. Cela signifie que selon l'invention les séquences SEQ ID n° 2 à SEQ ID n° 8 sont incluses dans la séquence SEQ ID n° 1.

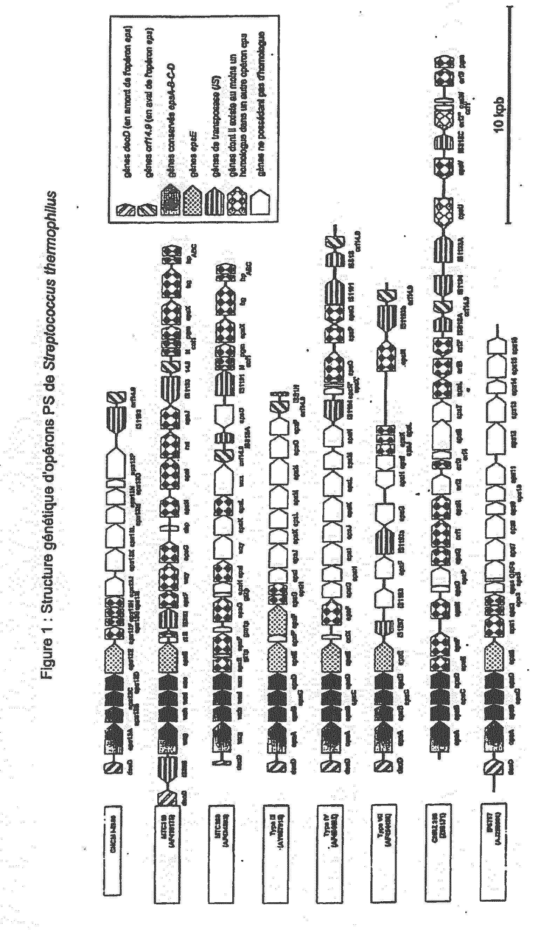

La structure de l'opéron selon l'invention a été déterminée (voir figure 1). Entre les gènes *deoD* (en amont de l'opéron PS) et *orf 14.9* (en aval), 17 ORF (open reading frame en anglais, ou cadre ouvert de lecture) dénommés *eps13A, eps13B, eps13C, eps13D, eps13E, eps13F, eps13G, eps13H, eps13I, eps13J, eps13K, eps13L, eps13M, eps13N, eps13O, eps13P* et *IS1193* ont été identifiés. Les 16 premiers ORF situés sur le brin "sens" codent potentiellement des polypeptides impliqués dans la production des polysaccharides exocellulaires ou capsulaires; le 17ème ORF situé sur le brin "antisens" code potentiellement une transposase fonctionnelle appartenant à la famille *IS1193* (séquence d'insertion).

Il est possible de dénommer chaque ORF et de le positionner. Ci-dessous sont indiquées la dénomination de chaque ORF, puis en second la fonction putative de la protéine déduite, et enfin en troisième la position de la région comprenant cet ORF (par rapport à la séquence SEQ ID n° 1 telle qu'indiquée dans la liste des séquences):
- *eps13A* : Régulateur transcriptionnel (342..1802)
- *eps13B :* Polymérisation (régulation de la longueur des chaînes) et/ou export des polysaccharides (1803..2534)
- *eps13C :* Polymérisation (régulation de la longueur des chaînes) et/ou export des polysaccharides (2543..3235)
- *eps13D :* Polymérisation (régulation de la longueur des chaînes) et/ou export des polysaccharides (3245..3985)
- *eps13E :* Undécaprenyl-phosphate glycosyltransférase (4042. 5409)
- *eps13F* : Undécaprenyl-phosphate glycosyltransférase (5611..6195)
- *eps13G* : Undécaprenyl-phosphate glycosyltransférase (6251..6634)
- *eps13H*: Beta-1,4-galactosyltransférase (6643..7092)
- *eps13I* : Beta-1,4-galactosyltransférase (7092..7607)
- *eps13J* : Rhamnosyltransférase (7597..8493)
- *eps13K* : Glycosyltransférase (8763..9797)
- *eps13L* : Polymérase d'unité répétitive (9827..10969)
- *eps13M* : Polymérase d'unité répétitive (10984..11793)
- *eps13N* : Glycosyltransférase (11844..12578)
- *eps13O*: Glycosyltransférase (12633..13016)
- *eps13P* : Transporteur transmembranaire (13049..14482)
- *IS1193* : Transposase (complément (14614..15870)).

L'invention concerne aussi un acide nucléique comprenant au moins une séquence sélectionnée parmi le groupe constitué par les séquences nucléotidiques SEQ ID n° 1, SEQ ID n° 2, SEQ ID n° 3, SEQ ID n° 4, SEQ ID n° 5, SEQ ID n°6, SEQ ID n° 7, SEQ ID n° 8.

Les séquences nucléotidiques SEQ ID n° 1, SECS ID n° 2, SET ID n° 3, SEQ ID n° 4, SECS ID n° 5, SEQ ID n°6, SEQ ID n° 7, SEQ ID n° 8 peuvent être insérées dans un vecteur par génie génétique, notamment par les techniques de l'ADN recombinant qui sont largement connues par l'homme du métier.

L'invention concerne également un vecteur de clonage et/ou d'expression comprenant au moins une séquence sélectionnée parmi le groupe constitué par les séquences nucléotidiques SEQ ID n° 1, SECS ID n° 2, SEQ ID n° 3, SEQ ID n° 4, SEQ ID n° 5, SEQ ID n° 6, SEQ ID n° 7, SEQ ID n° 8 ou un acide nucléique tel que défini ci-dessus.

Le vecteur préféré selon l'invention est un plasmide. Il peut s'agir d'un plasmide réplicatif ou intégratif.

A partir de ces vecteurs et/ou plasmides il est possible de transformer une bactérie afin d'y inclure ces vecteurs et/ou plasmides. Cette transformation peut être réalisée par la technique d'électroporation ou par conjugaison, et ceci de manière classique pour l'homme du métier.

L'invention concerne aussi des bactéries hôtes transformées par un plasmide de clonage ou d'expression ou un plasmide de clonage ou d'expression comprenant la séquence nucléotidique SEQ ID n° 1.

De manière préférée les bactéries transformées sont des bactéries lactiques. En particulier il s'agit d'une bactérie lactique qui peut être choisie parmi les genres *Streptococcus, Lactococcus, Lactobacillus, Leuconostoc, Pediococcus* et *Bifidobacterium.*

La souche de bactérie lactique préférée selon l'invention est *Streptococcus thermophilus.*

L'invention concerne aussi un procédé de construction d'une souche ou d'une bactérie hôte transformée selon l'invention caractérisé en ce qu'elles sont obtenues par transformation à l'aide d'un vecteur comprenant au moins une séquence sélectionnée parmi le groupe constitué par les séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8, ou avec un acide nucléique comprenant au moins une séquence sélectionnée parmi le groupe constitué par les séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8.

Selon le procédé de l'invention, le vecteur préféré est un plasmide.

Avantageusement selon le procédé de l'invention la transformation est suivie d'une insertion dans le génome de la souche ou de la bactérie hôte transformée par au moins un événement de recombinaison.

L'invention concerne aussi une composition bactérienne comprenant au moins une souche selon l'invention ou comprenant au moins une bactérie transformée selon l'invention.

Par composition bactérienne on entend un mélange de différentes souches, notamment un ferment, ou un levain.

Les mélanges de souches préférés selon l'invention sont des mélanges de *Streptococcus thermophilus* avec d'autres *Streptococcus thermophilus,* ou des mélanges de *Streptococcus thermophilus* avec *Lactobacillus delbrueckil subsp. Bulgaricus,* ou des mélanges de *Streptococcus thermophilus* avec d'autres *Lactobacillus* et/ou avec *Bifidobacterium,* ou des mélanges de *Streptococcus thermophilus* avec d'autres *Lactococcus,* ou des mélanges de *Streptococcus thermophilus* avec d'autres souches de bactéries lactiques et/ou de levures.

L'invention concerne aussi utilisation d'une souche de bactérie lactique du genre *Streptococcus* qui comprend au moins une séquence sélectionnée parmi le groupe constitué par les séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8 ou d'une souche selon la revendication 1 ou d'une souche obtenue selon le procédé des revendications 16 à 18 où la souche est choisie parmi le genre *Streptococcus* pour fabriquer un produit alimentaire ou un ingrédient alimentaire pour texturer un produit alimentaire.

A titre de produit alimentaire ou d'ingrédient alimentaire préféré selon l'invention on peut citer un produit laitier, un produit carné, un produit céréalier, une boisson, une mousse ou une poudre.

Des compositions alimentaires ou pharmaceutiques comprenant au moins une souche selon l'invention ou au moins une souche obtenue selon le procédé de l'invention ou la composition bactérienne selon l'invention sont également décrites.

Un produit laitier comprenant au moins une souche selon l'invention ou au moins une souche obtenue selon le procédé de l'invention ou la composition bactérienne selon l'invention est également décrite.

Dans le cas de la fabrication d'un produit laitier, celle-ci sera réalisée de manière habituelle dans ce domaine, et notamment par fermentation d'un produit laitier par incorporation d'une souche selon l'invention.

A titre de produit laitier selon l'invention, on peut citer un lait fermente, un yaourt, une crème maturée, un fromage, un fromage frais, une boisson lactée, un rétentat de produit laitier, un fromage fondu, une crème dessert, un fromage du cottage ou un lait infantile.

De préférence le produit laitier selon l'invention comprend un lait d'origine animale et/ou végétale.

A titre de lait d'origine animale on peut citer le lait de vache, de brebis, de chèvre, ou de bufflesse.

A titre de lait d'origine végétale on peut citer toute substance fermentescible d'origine végétale qui peut être utilisée selon l'invention notamment provenant de graine de soja, de riz ou de malt.
La figure 1 représente la structure génétique de l'opéron PS de la souche selon l'invention comparée à l'éperon PS d'autres souches de *Streptococcus thermophilus* déjà connues.
La figure 2 représente le plan factoriel 1-2 de l'analyse en composantes principales (ACP) obtenu à partir des données sensorielles sur les laits fermentes obtenus avec les différentes souches de l'étude.
La figure 3 représente les moyennes des notes obtenues sur les descripteurs de TEXTURE A LA CUILLERE pour chacune des souches de l'étude. Interprétation des résultats du test de Newman-Keuls : la différence entre les souches reliées par une même lettre n'est pas significative.
La figure 4 représente les moyennes des notes obtenues sur les descripteurs de TEXTURE EN BOUCHE pour chacune des souches de l'étude. Interprétation des résultats du test de Newman-Keuls : la différence entre les souches reliées par une même lettre n'est pas significative.

Des exemples concrets mais non limitatifs de l'invention vont maintenant être décrits.

### EXEMPLES

### 1/ Séquençage de l'opéron PS de Streptococcus thermophilus CNCM I-2980

Le fragment d'ADN portant l'opéron PS de la souche *Streptococcus thermophilus* CNCM I-2980 a été obtenu par amplification PCR (polymerase chain reaction, en anglais) à partir de l'ADN génomique extrait de ladite souche. L'amplification a été réalisée avec le thermocycleur Mastercycler (Eppendorf) en utilisant l'ADN polymérase LA-*Taq* (BioWhittaker/Cambrex) et les amorces suivantes : 5'-GGGTGAACGTATCTCAGTAATGGGGACTGG-3' et 5'-CCTGAGTTATGGGACGATTACTTGGCTG-3'. Les conditions expérimentales de cette amplification sont les suivantes : 14 cycles alternant dénaturation à 98°C pendant 30 s et hybridation-élongation à 68°C pendant 15 min, puis 16 cycles alternant dénaturation à 98°C pendant 30 s et hybridation-élongation à 68°C pendant 15 min avec un incrément de 15 s par cycle, puis 1 cycle supplémentaire d'élongation à 72°C pendant 10 min. Le produit de PCR a été séquencé selon une approche de clonage par fragments.

### 2/ Caractérisation moléculaire de la souche selon l'invention comparée avec des souches de Streptococcus thermophilus déjà connues

### O Séquence de l'opéron PS

La séquence de l'opéron PS de la souche *Streptococcus thermophilus* CNCM I-2980 a été obtenue à partir d'un fragment d'ADN d'environ 17100 paires de bases synthétisé par PCR en présence d'une matrice d'ADN génomique purifié de la souche et en utilisant deux amorces spécifiques de gènes conservés (*deoD* codant une purine-nucléotide phosphorylase, et *orf14.9* de fonction inconnue) encadrant généralement l'opéron PS chez les *Streptococcus thermophilus* décrits dans la littérature. La séquence comprise entre les gènes *deoD* et *orf14.9,* correspondant à la séquence SEQ ID n°1, est donnée avec la liste des séquences.

### • Organisation génétique de l'opéron PS

La figure 1 schématise la structure génétique de l'opéron PS de la souche CNCM I-2980 établie par analyse de sa séquence nucléotidique, ainsi que la structure de l'opéron PS de 7 autres souches de *Streptococcus thermophilus.* Pour les différentes structures, identifiées par le nom de la souche et par le numéro d'accès GENBANK (entre parenthèses), les flèches représentent la position, la taille et l'orientation des gènes depuis le codon d'initiation jusqu'au codon stop. La signification des couleurs et/ou motifs à l'intérieur des flèches est donnée par la légende de la figure 1.

### • Comparaison avec la littérature

L'analyse structurale de l'opéron PS de la souche CNCM I-2980 montre qu'il possède une organisation globale similaire à celle des opérons PS déjà connus (voir figure 1) : un premier ORF potentiellement impliqué dans la régulation de la transcription de l'opéron PS, suivi de 3 ORF intervenant probablement dans la régulation de la polymérisation des unités répétitives du PS et/ou leur export, suivis de 11 ORF codant des glycosyltransférases et une polymérase assurant l'assemblage de l'unité répétitive, eux-mêmes suivis d'1 ORF potentiellement impliqué dans le transport des unités répétitives à travers la membrane plasmidique.

L'environnement génétique de l'opéron PS de la souche CNCM I-2980 est également similaire à celui d'autres opérons PS connus : en amont l'ORF *deoD* codant une purine-nucléotide phosphorylase, et en aval dans le sens opposé les ORF *IS1193* et *orf14.9* codant respectivement une transposase (appartenant à la famille des séquences d'insertion *IS1193,* éléments génétiques mobiles) et une protéine de fonction inconnue.

Cependant, la comparaison de séquence réalisée entre les protéines potentiellement codées par les ORF de l'opéron de la souche CNCM I-2980 et celles disponibles dans les bases publiques de données (GENBANK) montre que le contenu génétique de l'opéron PS de la souche CNCM I-2980 est nouveau dans sa partie distale.
- La partie proximale de l'opéron PS des *Streptococcus thermophilus,* et plus généralement des streptocoques connus à ce jour, comprend 4 ORF appelés *epsA* (ou *cpsA*, ou *capA,* ou *wzg*), *epsB* (ou *cpsB*, ou *capB*, ou *wzh*), *epsC* (ou *cpsC,* ou *cpaC,* ou *wzd*) et *epsD* (ou *cpsD,* ou *capD,* ou *wze*) dont les produits polypeptidiques déduits, pour chacun des 4 ORF, présentent d'importantes similitudes de séquence entre souches. A ce niveau, les produits polypeptidiques déduits des ORF *eps13A, eps13B, eps13C* et *eps13D* de l'opéron PS de la souche CNCM I-2980 ne se distinguent pas de ceux déduits des autres opérons PS (pourcentage d'acides aminés identiques supérieur ou égal à 94%).
- Suivant immédiatement l'ORF *epsD,* l'ORF *epsE* (ou *cpsE,* ou *capE,* ou *wchA*) est présent dans la majorité des opérons PS connus chez *Streptococcus thermophilus.* Dans ce cas aussi, les similitudes de séquence sont importantes entre produits polypeptidiques homologues issus des différentes séquences connues ; le produit de traduction de l'ORF *eps13E* de la souche CNCM I-2980 est fortement similaire à ses homologues issus d'autres souches.
- L'organisation des 4 ORF suivants (*eps13F, eps13G, eps13H* et *eps13I*) de l'opéron PS de la souche CNCM I-2980, bien que déjà décrite, est moins fréquente parmi les différentes structures connues d'opérons PS de *Streptococcus thermophilus.* Cette organisation est trouvée, parfois incomplètement, dans les souches IP6757 (numéro d'accès GENBANK AJ289861), "type VII" (numéro d'accès GENBANK AF454498) et "type III" (numéro d'accès GENBANK AY057915).
- Dans la partie distale de l'opéron, les 7 ORF *eps13J, eps13K, eps13L, eps13M, eps13N, eps13O* et *eps13P* sont nouveaux et spécifiques de l'opéron PS de la souche CNCM I-2980. Bien que faible, leur similitude de séquence au niveau protéique, ou dans certains cas l'existence de motifs protéiques spécifiques, permet d'assigner une fonction putative aux produits de ces ORF : activité potentielle de glycosyltransférase ou polymérase pour le produit des ORF *eps13J* à *eps13O,* activité de transport transmembranaire du PS pour le produit de l'ORF *eps13P.*

### 3/ Test comparé de rhéologie de la souche Streptococcus thermophilus CNCM I-2980

Les souches utilisées de *Streptococcus thermophilus* CNCM I-2423, CNCM I-2429, CNCM I-2432, CNCM I-2978 et CNCM 1-2979 sont des souches de collection de Rhodia Food. Elles sont pour la plupart utilisées pour la production industrielle de laits fermentés ou de yaourts et reconnues pour leurs propriétés texturantes. Elles sont représentatives des souches décrites dans la littérature. Elles sont étudiées par la suite comparativement à la souche CNCM I-2980 et considérées comme représentatives des souches texturantes utilisées actuellement dans l'industrie agroalimentaire.

Les souches de *Streptococcus thermophilus* RD736 et RD676 sont des souches industrielles de Rhodia Food réputées pour leur faible pouvoir texturant. Les polysaccharides qu'elles pourraient produire et leur opéron PS ne sont pas connus. Elles sont étudiées par la suite comparativement à la souche CNCM I-2980 et considérées comme représentatives des souches non texturantes.

Le lait fermenté utilisé est obtenu en supplémentant 100 ml de lait UHT ½ écrémé (Le Petit Vendéen®) par 3% (poids/volume) de poudre de lait écrémé. La stérilité de la solution est obtenue par une pasteurisation à 90°C pendant 10 min, la température est mesurée à coeur du lait. Le support de fermentation ainsi obtenu est inoculé avec la souche à tester à raison de 10E+6 ufc/ml (unité formant colonie/ml) puis incubé à 43°C (au bain-marie) jusqu'à l'obtention d'un pH de 4,6. Le suivi du pH est enregistré en continu. Les laits fermentés ainsi obtenus sont placés dans une étuve ventilée à 6°C, jusqu'à leur analyse.

Deux types de mesures rhéologiques sont effectués : viscosité et écoulement. Les mesures de viscosité sont réalisées à 8°C sur des laits fermentés, après 1, 7, 14 et 28 jours de stockage à 6°C. L'appareillage utilisé est un viscosimètre Brookfield® de type RVF (Brookfield Engineering Laboratories, Inc.) monté sur pied Helipath (Brookfield Engineering Laboratories, Inc.). Le viscosimètre est équipé d'une aiguille de type C et la vitesse d'oscillation appliquée à l'aiguille est de 10 tours/min. Les mesures d'écoulement sont effectuées à 8°C sur des laits fermentés, après 14 jours de stockage à 6°C et qui ont été préalablement brassés. L'appareillage utilisé est un rhéomètre AR1000-N (TA Instrument) équipé de cylindres co-axiaux (Rayon 1 = 15 mm, Rayon 2 = 13,83 mm, Hauteur = 32 mm, Entrefer = 2 mm). Pour le segment de montée, la contrainte appliquée en balayage continu varie de 0 à 60 Pa pendant une durée de 1 min selon un mode linéaire. Pour le segment de descente, la contrainte appliquée en balayage continu varie de 60 à 0 Pa pendant une durée de 1 min selon un mode linéaire. Les valeurs prises en compte sont l'aire de thixotropie et le seuil d'écoulement ; ce dernier est calculé selon le modèle de Casson.

La viscosité du lait fermenté obtenu avec la souche *Streptococcus thermophilus* CNCM I-2980 a été mesurée après 1, 7, 14 et 28 jours de stockage à 6°C (tableau 1). La valeur de viscosité mesurée après une journée de stockage est de 53,3 Pa·s. Cette valeur varie peu dans le temps montrant la stabilité du lait fermenté obtenu avec la souche *Streptococcus thermophilus* CNCM I-2980. Comparativement (tableau 1), les viscosités mesurées pour les laits fermentés fabriqués avec les souches RD736 et RD676, réputées à faible pouvoir texturant, sont comprises entre 26 et 30 Pa.s. Les autres souches testées génèrent des laits fermentés présentant des viscosités plus faibles que celle obtenue avec la souche CNCM I-2980. On peut distinguer un groupe de souches générant des viscosités de l'ordre de 40 Pa.s (CNCM I-2979, CNCM I-2423 et CNCM I-2432) et un second, auquel appartient CNCM I-2980, générant des viscosités de l'ordre de 50 Pa.s.

**Tableau 1 : Viscosité et pH des laits fermentés obtenus avec les différentes souches testées, après différentes durées de stockage à 6°C.**

| **Souches** | **Viscosité en Pa·s / pH** | | | |
|---|---|---|---|---|
| | Stockage 1 jour | Stockage 7 jours | Stockage 14 jours | Stockage 28 jours |
| **CNCM I-2980** | 53,3 / 4,50 | 53,0 / 4,44 | 53,0 / 4,42 | 53,5 / 4,40 |
| **CNCM I-2429** | 51,2 / 4,55 | 51,9 / 4,45 | 51,0 / 4,44 | 59,2 / 4,44 |
| **CNCM I-2978** | 50,4 / 4,56 | 51,8 / 4,52 | 49,6 / 4,49 | 51,0 / 4,45 |
| **CNCM I-2432** | 42,4 / 4,60 | 42,2 / 4,56 | 43,0 / 4,55 | 43,0 / 4,45 |
| **CNCM I-2423** | 42,0 / 4,60 | 41,9 / 4,40 | 42,0 / 4,37 | 43,0 / 4,30 |
| **CNCM I-2979** | 37,8 / 4,54 | 40,7 / 4,45 | 42,2 / 4,42 | 42,2 / 4,33 |
| **RD676** | 29,6 / 4,60 | 30,0 / 4,57 | 30,0 / 4,57 | 30,0 / 4,52 |
| **RD736** | 26,0 / 4,45 | 26,0 / 4,34 | 28,0 / 4,34 | 27,0 / 4,26 |

Les mesures d'écoulement ont permis de définir deux descripteurs rhéologiques significatifs (seuil d'écoulement et aire de thixotropie) pour la description rhéologique des laits fermentés (tableau 2). Pour le lait fermenté obtenu avec la souche CNCM I-2980, les valeurs moyennes sont de 5,89 Pa et de 488 Pa/s pour, respectivement, le seuil d'écoulement et l'aire de thixotropie. Ces valeurs sont significativement différentes de celles obtenues pour les laits fermentés obtenus avec les souches réputées non texturantes (RD676 et RD736). Par exemple pour le lait fermenté obtenu avec la souche RD676, ces valeurs moyennes sont respectivement de 17,01 Pa et de 17083 Pa/s. Dans le cas des souches réputées texturantes, les valeurs de seuil d'écoulement et d'aire de thixotropie sont beaucoup plus proches de celles obtenues avec la souche CNCM I-2980, mais significativement supérieures cependant, montrant une aptitude texturante supérieure de la souche CNCM I-2980.

**Tableau 2 : Valeurs de seuil d'écoulement et d'aire de thixotropie (moyenne de 3 répétitions) mesurées à l'aide de l'appareil AR1000-N des laits fermentés obtenus avec les différentes souches testées après 14 jours de stockage à 6°C.**

| **Souches** | **Seuil d'écoulement (Pa)** | **Aire de thixotropie (Pa/s)** | |
|---|---|---|---|
| **CNCM I-2980** | 5,89 | 488 | |
| **CNCM I-2429** | 13,32 | 1215 | (2 valeurs) |
| **CNCM I-2978** | 10,51 | 728 | |
| **CNCM I-2432** | 12,27 | 1245 | |
| **CNCM I-2423** | 8,86 | 1344 | |
| **CNCM I-2979** | 13,56 | 1786 | |
| **RD676** | 17,01 | 17083 | |
| **RD736** | 15,91 | 33100 | |

### 4/ Caractérisation sensorielle de la souche Streptococcus thermophilus CNCM I-2980, comparaison avec les souches de référence

Les laits fermentés ont été évalués en analyse sensorielle après 14 jours de stockage à 6°C. L'analyse descriptive quantitative des laits fermentés, maintenus à une température optimale de dégustation de 12°C, a été réalisée par un jury de 9 experts sur une échelle linéaire non structurée de 0 à 6 points. Cette analyse de profil sensoriel a été dupliquée à quelques jours d'intervalle. Les juges, préalablement sélectionnés et entraînés, ont effectué leur évaluation sur 4 descripteurs de texture à la cuillère : cassant, résistance au brassage, filant, granulosité, et sur 4 descripteurs de texture en bouche : fondant, collant, épaisseur, nappant. Les différences sensorielles ont été évaluées par une analyse de variance (dénommée ANOVA) à deux facteurs, modèle fixe, suivie d'un test de comparaison de moyenne de Newman-Keuls avec un seuil alpha de 5% sur chacun des descripteurs. Une Analyse en Composantes Principales (ACP) avec les descripteurs sensoriels en variables et les souches en individus a été mise en oeuvre pour visualiser l'espace produit. Une Classification Ascendante Hiérarchique (CAH) a permis de dégager les groupes de souches sur l'ACP. Les logiciels utilisés pour ces analyses statistiques sont Fizz® (Biosystèmes), Statgraphics® et Uniwin plus®.

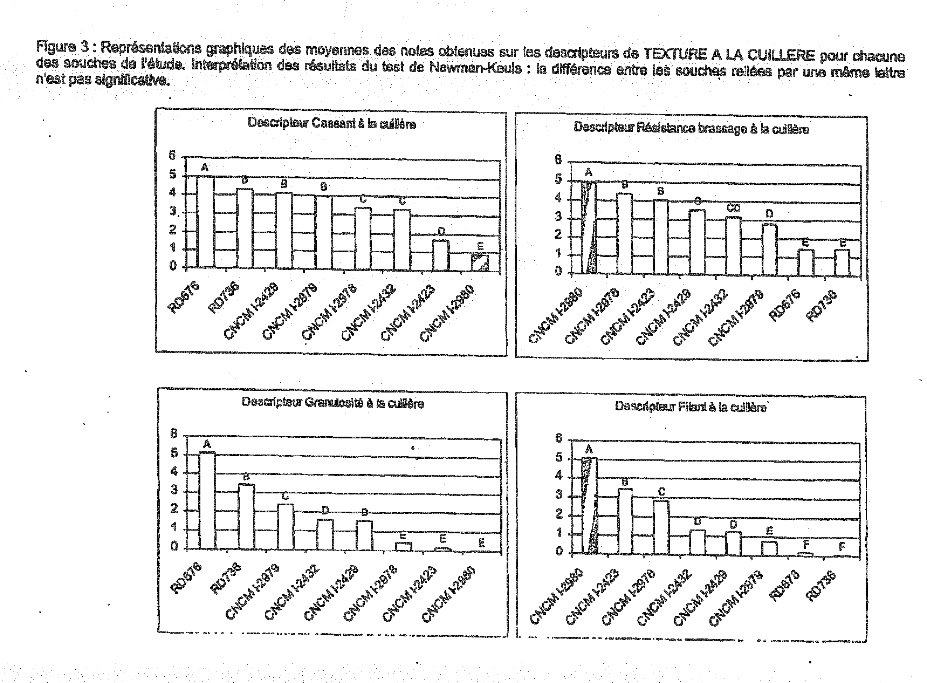

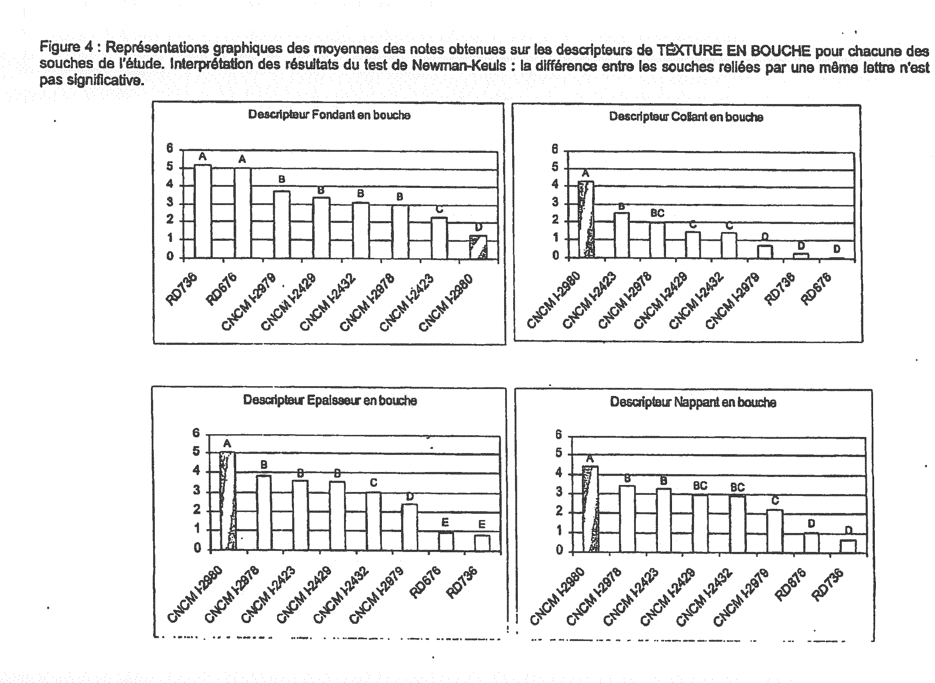

Les données du lait fermenté obtenu avec la souche *Streptococcus thermophilus* CNCM I-2980 ont été confrontées à celles des laits fermentés obtenus avec les autres souches de référence. Les valeurs moyennes obtenues avec les différentes souches pour les descripteurs de texture retenus sont indiquées dans le tableau 3 et les différences significatives ressortant de l'ANOVA et du test de comparaison de moyennes sont répertoriées dans le tableau 4 et les figures 3 et 4.

**Tableau 3 : Moyenne des notes attribuées par le jury d'analyse sensorielle pour les laits fermentés obtenus avec les différentes souches de l'étude sur les descripteurs de texture.**

| **Souches** | **Descripteurs à la cuillère** | | | | **Descripteurs en bouche** | | | |
|---|---|---|---|---|---|---|---|---|
| | **Cassant** | **Résistance au brassage** | **Granuleux** | **Filant** | **Fondant** | **Collant** | **Epaisseur** | **Nappant** |
| **CNCM I-2980** | 0,89 | 4,97 | 0,04 | 5,09 | 1,32 | 4,27 | 5,06 | 4,41 |
| **CNCM I-2429** | 4,12 | 3,49 | 1,53 | 1,24 | 3,36 | 1,46 | 3,54 | 2,94 |
| **CNCM I-2978** | 3,39 | 4,35 | 0,42 | 2,83 | 2,96 | 1,97 | 3,86 | 3,41 |
| **CNCM I-2432** | 3,28 | 3,14 | 1,60 | 1,32 | 3,11 | 1,43 | 3,01 | 2,91 |
| **CNCM I-2423** | 1,64 | 4,02 | 0,16 | 3,44 | 2,25 | 2,48 | 3,61 | 3,30 |
| **CNCM I-2979** | 3,98 | 2,79 | 2,36 | 0,74 | 3,71 | 0,72 | 2,39 | 2,22 |
| **RD676** | 4,98 | 1,46 | 5,14 | 0,17 | 4,98 | 0,04 | 0,91 | 1,03 |
| **RD736** | 4,35 | 1,45 | 3,43 | 0,04 | 5,18 | 0,31 | 0,77 | 0,67 |

**Tableau 4 : Comparaison de moyennes sur chacun des descripteurs de texture à la cuillère et de texture en bouche par le test de Newman-Keuls à 5%. Interprétation des résultats : la différence entre les souches reliées par une même lettre n'est pas significative.**

| **Souches** | **Descripteurs à la cuillère** | | | | **Descripteurs en bouche** | | | |
|---|---|---|---|---|---|---|---|---|
| | **Cassant** | **Résistance au brassage** | **Granuleux** | **Filant** | **Fondant** | **Collant** | **Epaisseur** | **Nappant** |
| **CNCM I-2980** | E | A | E | A | D | A | A | A |
| **CNCM I-2429** | B | C | D | D | B | C | B | BC |
| **CNCM I-2978** | C | B | E | C | B | BC | B | B |
| **CNCM I-2432** | C | CD | D | D | B | C | C | BC |
| **CNCM I-2423** | D | B | E | B | C | B | B | B |
| **CNCM I-2979** | B | D | C | E | B | D | D | C |
| **RD676** | A | E | A | F | A | D | E | D |
| **RD736** | B | E | B | F | A | D | E | D |

Les figures 3 et 4 montrent une représentation par histogramme des résulats obtenus dans le tableau 4.

Pour tous les descripteurs, les souches considérées non texturantes RD676 et RD736 se démarquent significativement des autres souches. Parmi les souches texturantes, la souche CNCM I-2980 se différencie nettement et significativement pour tous les descripteurs, excepté pour la granulosité où elle n'est pas différentiable des souches CNCM I-2978 et CNCM I-2423.

L'ACP permet de positionner les souches en fonction de leur distance par rapport aux descripteurs sensoriels.

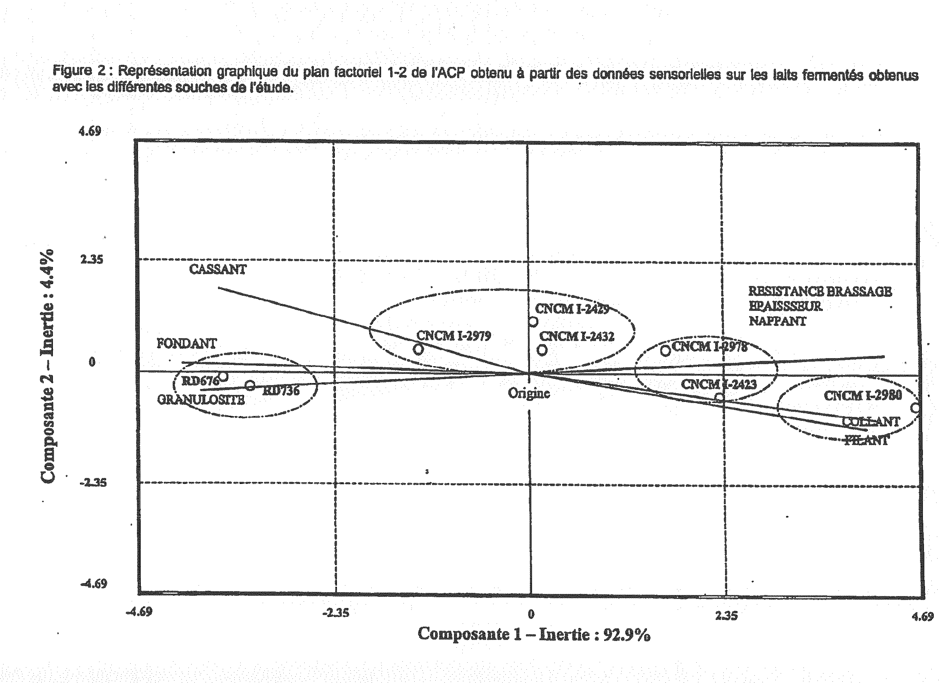

Le plan 1-2 de l'ACP en figure 2 représente 97,3% de l'espace produit. La composante 1 oppose deux groupes de variables sensorielles. Le premier groupe, constitué des variables : résistance au brassage, épaisseur en bouche, nappant en bouche, filant à la cuillère et collant en bouche, explique la composante 1 à droite. Le second groupe, constitué des variables : fondant en bouche, cassant à la cuillère et granulosité à la cuillère, explique la composante 1 à gauche. Le premier groupe de variables est anti-corrélé au second groupe. Ces variables permettent d'analyser le positionnement des souches sur ce plan. De plus, l'analyse en CAH permet de classer les souches en différents groupes qui sont représentés sous forme de cercles en pointillé sur le plan factoriel 1-2.

Le plan factoriel oppose plusieurs groupes de souches apportant des propriétés de texture différentes. Il ressort de ces analyses que les souches RD736 et RD676 confèrent au lait fermenté une texture cassante et granuleuse à la cuillère et fondante en bouche. De la même façon, elles ne donnent pas une texture épaisse, collante ou nappante en bouche, ni résistante ou filante à la cuillère comparativement aux autres groupes de souches. Elles donnent donc un lait fermenté non texturé. Par opposition, les souches CNCM I-2429, CNCM I-2432 et CNCM I-2979 donnent des laits fermentés moyennement texturés ; les souches CNCM I-2423 et CNCM I-2978 donnent des laits fermentés texturés, et la souche CNCM I-2980 donne un lait fermenté fortement texturé. Cette analyse montre que la souche CNCM I-2980 apporte des caractéristiques de texture bien particulières dans le lait fermenté comparativement à l'ensemble des souches de référence.

### 5/ Test comparatif de résistance de la souche Streptococcus thermophilus CNCM I-2980 aux phages

La sensibilité d'une souche à un bactériophage est établie par la méthode des plages de lyse. Cent µl d'une culture de la souche à tester et 100 µl d'une dilution appropriée d'un sérum contenant le bactériophage à étudier sont utilisés pour ensemencer 5 ml d'un milieu gélosé en surfusion (agar 0,6% poids/volume) M17 glucose supplémenté à 10 mM en CaCl₂. L'ensemble est versé à la surface d'un milieu gélosé solidifié (agar 1,5% poids/volume) M17 glucose supplémenté à 10 mM en CaCl₂. Après incubation à 42°C pendant 16 heures, la sensibilité de la souche au bactériophage est évaluée par la présence de plages de lyse. L'absence de plage de lyse signifie la résistance de cette souche aux bactériophages testés. Le spectre de sensibilité d'une souche aux bactériophages (aussi appelé lysotype) est constitué par l'ensemble des sensibilités et résistances aux bactériophages étudiés.

Le tableau 5 ci-dessous définit les bactériophages utilisés pour cette étude et leur souche d'origine/de propagation. Il s'agit de souches et de phages issus de la collection de Rhodia Food. Les bactériophages ont été sélectionnés pour leur pouvoir infectieux des souches texturantes de référence.

**Tableau 5 : Phages**

| **Nom du phage** | **Souche d'origine** |
|---|---|
| 2972 | CNCM I-2423 |
| 4082 | RD729 |
| 4074 | CNCM I-2429 |
| 4154 | CNCM I-2429 |
| 1272 | CNCM I-2978 |
| 4128 | RD852 |
| 1255 | CNCM I-2432 |
| 1765 | RD728 |
| 4121 | RD862 |

Pour évaluer l'intérêt industriel de la souche CNCM I-2980 par rapport aux problèmes associés aux bactériophages, le lysotype de la souche CNCM I-2980 a été évalué et comparé à ceux des souches texturantes de référence. Le tableau 6 visualise les sensibilités des souches à ces différents phages (lysotype) établies par la méthode des plages de lyse. Il apparaît que les six souches de l'étude ont des lysotypes différents. En particulier, la souche CNCM I-2980 présente un lysotype distinct de celui des autres souches texturantes testées. En effet la souche CNCM I-2980 n'a pas pu être infectée par les phages testés.

**Tableau 6 : Lysotype des souches testées**

| **Phages** | **Souches** | | | | | |
|---|---|---|---|---|---|---|
| | CNCM I-2980 | CNCM I-2423 | CNCM I-2978 | CNCM I-2432 | CNCM I-2429 | CNCM I-2979 |
| 2972 | - | + | - | - | - | - |
| 4082 | - | + | - | - | - | - |
| 1272 | - | - | + | - | - | - |
| 4128 | - | - | + | - | - | - |
| 1255 | - | - | - | + | - | - |
| 1765 | - | - | - | + | - | - |
| 4074 | - | - | - | - | + | - |
| 4154 | - | - | - | - | + | - |
| 4121 | - | - | - | - | - | + |
| + : sensible au phage testé ; - : résistante au phage testé | | | | | | |

## Revendications

1. Souche de *Streptococcus thermophilus* déposée le 26 février 2003 à la Collection Nationale de Cultures de Microorganismes sous le numéro I-2980.

2. Séquence nucléotidique SEQ ID n° 1.

3. Séquence nucléotidique SEQ ID n° 2.

4. Séquence nucléotidique SEQ ID n° 3.

5. Séquence nucléotidique SEQ ID n° 4.

6. Séquence nucléotidique SEQ ID n° 5.

7. Séquence nucléotidique SEQ ID n° 6.

8. Séquence nucléotidique SEQ ID n° 7.

9. Séquence nucléotidique SEQ ID n° 8.

10. Acide nucléique comprenant au moins une séquence sélectionnée parmi le groupe constitué par les séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8.

11. Vecteur de clonage et/ou d'expression comprenant au moins une séquence sélectionnée parmi le groupe constitué par les séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8 ou un acide nucléique selon la revendication 10.

12. Vecteur selon la revendication 11 **caractérisé en ce qu'**il s'agit d'un plasmide.

13. Bactérie hôte transformée par un vecteur de clonage ou d'expression ou un plasmide de clonage ou d'expression comprenant la séquence nucléotidique SEQ ID n° 1.

14. Bactérie selon la revendication 13 **caractérisée en ce qu'**il s'agit d'une bactérie lactique.

15. Bactérie selon la revendication 13 ou 14 **caractérisée en ce qu'**il s'agit d'une bactérie lactique choisie parmi les genres *Streptococcus, Lactococcus, Lactobacillus, Leuconostoc, Pediococcus* et *Bifidobacterium.*

16. Procédé de construction d'une souche selon la revendication 1 ou d'une bactérie selon les revendications 13 à 15 **caractérisé en ce que** la souche ou la bactérie sont obtenues par transformation à l'aide d'un vecteur comprenant au moins une séquence sélectionnée parmi le groupe constitué par les séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8 ou un acide nucléique selon la revendication 10.

17. Procédé selon la revendication 16 **caractérisé en ce que** la transformation est suivie d'une insertion dans le génome de la souche ou de la bactérie par au moins un événement de recombinaison.

18. Procédé selon la revendication 16 ou 17 **caractérisé en ce que** le vecteur est un plasmide.

19. Composition bactérienne comprenant au moins une souche selon la revendication 1 ou au moins une bactérie transformée selon les revendications 13 à 15.

20. Utilisation d'une souche de bactérie lactique du genre *Streptococcus* qui comprend au moins une séquence sélectionnée parmi le groupe constitué par les séquences nucléotidiques SEQ ID n° 1, SEQ ID n° 2, SEQ ID n° 3, SEQ ID n° 4, SEQ ID n° 5, SEQ ID n° 6, SEQ ID n° 7, SEQ ID n° 8 ou d'une souche selon la revendication 1 ou d'une souche obtenue selon le procédé des revendications 16 à 18 où la souche est choisie parmi le genre *Streptococcus* pour fabriquer un produit alimentaire ou un ingrédient alimentaire pour texturer un produit alimentaire.

21. Utilisation selon la revendication 20 **caractérisée en ce que** le produit alimentaire ou l'ingrédient alimentaire est un produit laitier, un produit carné, un produit céréalier, une boisson, une mousse ou une poudre.

22. Utilisation selon la revendication 21 où le produit laitier est un lait fermenté, un yaourt, une crème maturée, un fromage, un fromage frais, une boisson lactée, un rétentat de produit laitier, un fromage fondu, une crème dessert, un fromage du cottage ou un lait infantile.

23. Utilisation selon la revendication 21 où le produit laitier comprend du lait d'origine animale et/ou végétale.

24. Utilisation selon la revendication 20 ou 24 **caractérisée en ce que** la souche appartient à l'espèce *Streptococcus thermophilus.*

## Patentansprüche

1. Streptococcus-termophilus-Stamm, der am 26. Februar 2003 bei der Collection Nationale de Cultures de Microorganismes unter der Nr. I-2980 hinterlegt worden ist.

2. Nukleotidsequenz SEQ ID NO: 1.

3. Nukleotidsequenz SEQ ID NO: 2.

4. Nukleotidsequenz SEQ ID NO: 3.

5. Nukleotidsequenz SEQ ID NO: 4.

6. Nukleotidsequenz SEQ ID NO: 5.

7. Nukleotidsequenz SEQ ID NO: 6.

8. Nukleotidsequenz SEQ ID NO: 7.

9. Nukleotidsequenz SEQ ID NO: 8.

10. Nukleinsäure, umfassend mindestens eine Sequenz, die aus der Gruppe ausgewählt ist, die durch die Nukleotidsequenzen SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 gebildet wird.

11. Klonierungs- und/oder Expressionsvektor, umfassend mindestens eine Sequenz, die aus der Gruppe ausgewählt ist, die durch die Nukleotidsequenzen SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 oder eine Nukleinsäure gemäss Anspruch 10 gebildet wird.

12. Vektor gemäss Anspruch 11, **dadurch gekennzeichnet, dass** es sich um ein Plasmid handelt.

13. Bakterienwirt, transformiert durch einen Klonierungs- oder Expressionsvektor oder ein Klonierungs- oder Expressionsplasmid, der die Nukleotidsequenz SEQ ID NO: 1 umfasst.

14. Bakterie gemäss Anspruch 13, **dadurch gekennzeichnet, dass** es sich um eine Milchsäurebakterie handelt.

15. Bakterie gemäss Anspruch 13 oder 14, **dadurch gekennzeichnet, dass** es sich um eine Milchsäurebakterie handelt, die aus den Gattungen Streptococcus, Lactococcus, Lactobacillus, Leuconostoc, Pediococcus und Bifidobacterium ausgewählt ist.

16. Verfahren zur Herstellung eines Stammes gemäss Anspruch 1 oder eines Bakteriums gemäss Ansprüchen 13 bis 15, **dadurch gekennzeichnet, dass** der Stamm oder das Bakterium durch Transformation mit Hilfe eines Vektors gewonnen werden, der mindestens eine Sequenz umfasst, die aus der Gruppe ausgewählt ist, die durch die Nukleotidsequenzen SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 oder eine Nukleinsäure gemäss Anspruch 10 gebildet wird.

17. Verfahren gemäss Anspruch 16, **dadurch gekennzeichnet, dass** die Transformation von einer Insertion ins Genom des Stammes oder des Bakteriums durch mindestens einen Rekombinationsvorgang gefolgt wird.

18. Verfahren gemäss Anspruch 16 oder 17, **dadurch gekennzeichnet, dass** der Vektor ein Plasmid ist.

19. Bakterielle Zusammensetzung, umfassend mindestens einen Stamm gemäss Anspruch 1 oder mindestens ein Bakterium, das gemäss den Ansprüchen 13 bis 15 transformiert wurde.

20. Verwendung eines Milchsäurebakterienstammes der Gattung Streptococcus, der mindestens eine Sequenz umfasst, die aus der Gruppe ausgewählt ist, die durch die Nukleotidsequenzen SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8 gebildet wird, oder eines Stammes gemäss Anspruch 1 oder eines Stammes, der gemäss dem Verfahren der Ansprüche 16 bis 18 gewonnen wurde, wobei der Stamm aus der Gattung Streptococcus ausgewählt wird, um ein Nahrungsmittelprodukt oder einen Nahrungsmittelinhaltsstoff zur Texturierung eines Nahrungsmittelprodukts herzustellen.

21. Verwendung gemäss Anspruch 20, **dadurch gekennzeichnet, dass** das Nahrungsmittelprodukt oder der Nahrungsmittelinhaltsstoff ein Milchprodukt, ein Fleischprodukt, ein Getreideprodukt, ein Getränk, eine Mousse oder ein Pulver ist.

22. Verwendung gemäss Anspruch 21, wobei es sich bei dem Milchprodukt um eine fermentierte Milch, einen Joghurt, eine reife Sahne, einen Käse, einen Frischkäse, ein Milchgetränk, ein Retentat eines Milchprodukts, einen Schmelzkäse, eine Dessertcreme, einen Hüttenkäse oder eine Babymilch handelt.

23. Verwendung gemäss Anspruch 21, wobei das Milchprodukt Milch tierischen und/oder pflanzlichen Ursprungs umfasst.

24. Verwendung gemäss Anspruch 20 oder 24, **dadurch gekennzeichnet, dass** er zur Spezies Streptococcus thermophilus gehört.

## Claims

1. Strain of *Streptococcus thermophilus* deposited on 26th February 2003 at the Collection Nationale de Cultures de Microorganismes under number 1-2980.

2. Nucleotide sequence SEQ ID No. 1.

3. Nucleotide sequence SEQ ID No. 2.

4. Nucleotide sequence SEQ ID No. 3.

5. Nucleotide sequence SEQ ID No. 4.

6. Nucleotide sequence SEQ ID No. 5.

7. Nucleotide sequence SEQ ID No. 6.

8. Nucleotide sequence SEQ ID No. 7.

9. Nucleotide sequence SEQ ID No. 8.

10. Nucleic acid comprising at least one sequence selected from the group constituted by the nucleotide sequences SEQ ID No. 1, SEQ ID No. 2, SEQ ID No. 3, SEQ ID No. 4, SEQ ID No. 5, SEQ ID No. 6, SEQ ID No. 7, SEQ ID No. 8.

11. Cloning and/or expression vector comprising at least one sequence selected from the group constituted by the nucleotide sequences SEQ ID No. 1, SEQ ID No. 2, SEQ ID No. 3, SEQ ID No. 4, SEQ ID No. 5, SEQ ID No. 6, SEQ ID No. 7, SEQ ID No. 8 or a nucleic acid according to claim 10.

12. Vector according to claim 11 **characterized in that** it is a plasmid.

13. Host bacterium transformed by a cloning or expression vector or a cloning or expression plasmid comprising the nucleotide sequence SEQ ID No. 1.

14. Bacterium according to claim 13 **characterized in that** it is a lactic acid bacterium.

15. Bacterium according to claim 13 or 14 **characterized in that** it is a lactic acid bacterium chosen from the genera *Streptococcus, Lactococcus, Lactobacillus, Leuconostoc, Pediococcus* and *Bifidobacterium.*

16. Process for constructing a strain according to claim 1 or a bacterium according to claims 13 to 15 **characterized in that** the strain or the bacterium is obtained by transformation using a vector comprising at least one sequence selected from the group constituted by the nucleotide sequences SEQ ID No. 1, SEQ ID No. 2, SEQ ID No. 3, SEQ ID No. 4, SEQ ID No. 5, SEQ ID No. 6, SEQ ID No. 7, SEQ ID No. 8 or a nucleic acid according to claim 10.

17. Process according to claim 16 **characterized in that** the transformation is followed by an insertion into the genome of the strain or of the bacterium by at least one recombination event.

18. Process according to claim 16 or 17 **characterized in that** the vector is a plasmid.

19. Bacterial composition comprising at least one strain according to claim 1 or at least one transformed bacterium according to claims 13 to 15.

20. Use of a strain of lactic acid bacterium of the genus *Streptococcus* which comprises at least one sequence selected from the group constituted by the nucleotide sequences SEQ ID No. 1, SEQ ID No. 2, SEQ ID No. 3, SEQ ID No. 4, SEQ ID No. 5, SEQ ID No. 6, SEQ ID No. 7, SEQ ID No. 8 or of a strain according to claim 1 or of a strain obtained according to the process of claims 16 to 18 where the strain is chosen from the genus *Streptococcus* for the manufacture of a food product or food ingredient in order to texture a food product.

21. Use according to claim 20 **characterized in that** the food product or food ingredient is a dairy product, a meat product, a cereal product, a beverage, a mousse, or a powder.

22. Use according to claim 21 where the dairy product is a fermented milk, a yogurt, a matured cream, a cheese, a fromage frais, a milk beverage, a dairy product retentate, a process cheese, a cream dessert, a cottage cheese or infant milk.

23. Use according to claim 21 where the dairy product comprises milk of animal and/or plant origin.

24. Use according to claim 20 or 24 **characterized in that** the strain belongs to the species *Streptococcus thermophilus.*
